(19) Europäisches Patentamt
European Patent Office
Office européen des brevets

(11) Veröffentlichungsnummer: **0 518 105 A1**

(12) **EUROPÄISCHE PATENTANMELDUNG**

(21) Anmeldenummer: **92108736.7**

(22) Anmeldetag: **23.05.92**

(51) Int. Cl.5: **C07D 211/90**, C07D 401/04, A61K 31/44, //C07D401/06, C07D413/04,C07D401/14, C07D413/14

(30) Priorität: **07.06.91 DE 4118707**

(43) Veröffentlichungstag der Anmeldung: **16.12.92 Patentblatt 92/51**

(84) Benannte Vertragsstaaten: **AT BE CH DE DK ES FR GB GR IT LI LU NL PT SE**

(71) Anmelder: **BAYER AG**

**W-5090 Leverkusen 1 Bayerwerk(DE)**

(72) Erfinder: **Stoltefuss, Jürgen, Dipl.-Ing.**
**Parkstrasse 20**
**W-5657 Haan 2(DE)**
Erfinder: **Franckowiak, Gerhard, Dr.**
**Henselweg 10**
**W-5600 Wuppertal 1(DE)**
Erfinder: **Böshagen, Horst, Dr.**
**Wiesenstrasse 4**
**W-5657 Haan(DE)**
Erfinder: **Bechem Martin, Dr.**
**Hans-Böckler-Strasse 102**
**W-5600 Wuppertal 1(DE)**
Erfinder: **Gross, Rainer, Prof.Dr.**
**Platzhofstrasse 23**
**W-5600 Wuppertal 1(DE)**
Erfinder: **Hebisch, Siegbert, Dr.**
**Richard-Seel-Weg 11**
**W-5600 Wuppertal 1(DE)**
Erfinder: **Hütter, Joachim, Dr.**
**Teschen-Sudberger-Strasse 13**
**W-5600 Wuppertal 12(DE)**
Erfinder: **Rounding, Howard-Paul, Dr.**
**Pahlkestrasse 15**
**W-5600 Wuppertal 1(DE)**
Erfinder: **Schramm, Matthias, Dr.**
**Humperdinkstrasse 15**
**W-5600 Leverkusen 1(DE)**

(54) **3-Formyl-1,4-dihydropyridine, Verfahren zu ihrer Herstellung und ihre Verwendung als Arzneimittel.**

(57) Die vorliegende Erfindung betrifft 3-Formyl-1,4-dihydropyridine der allgemeinen Formel I

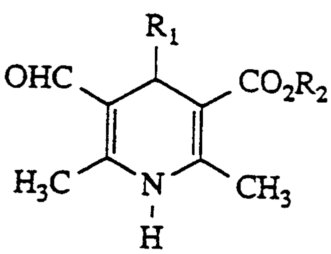

(I)

in welcher

$R^1$ für Phenyl steht, das gegebenenfalls bis zu 3-fach gleich oder verschieden durch Nitro, Cyano, Halogen, Trifuormethyl, Trifluormethylthio, Trifluormethoxy, Difluormethoxy oder durch geradkettiges oder verzweigtes Alkyl, Alkoxy, Alkylthio oder Alkoxycarbonyl mit jeweils bis zu 8 Kohlenstoffatomen, oder durch eine Gruppe der Formel $-O(CH_2)_n-R^3$, $-S-(CH_2)_n-R^3$ oder $-CO-(CH_2)_n-R^3$ substituiert ist,

EP 0 518 105 A1

worin

n eine Zahl 0,1,2,3 oder 4 bedeutet,

$R^3$ Aryl mit 6 bis 10 Kohlenstoffatomen bedeutet, das gegebenenfalls bis zu 3-fach gleich oder verschieden durch Halogen, Nitro, Trifluormethyl, Trifluormethoxy, Difluormethoxy, Trifluormethylthio oder durch geradkettiges oder verzeigtes Alkyl oder Alkoxy mit jeweils bis zu 8 Kohlenstoffatomen substituiert ist,

oder für einen Rest der Formel

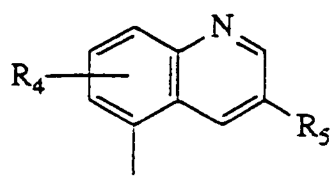

,

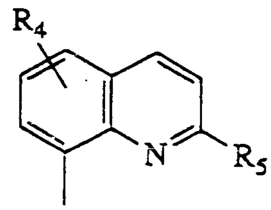

steht,

worin

$R^4$ Wasserstoff, Halogen oder geradkettiges oder verzweigtes Alkyl oder Alkoxy mit jeweils bis zu 8 Kohlenstoffatomen bedeutet,

$R^5$ Phenyl bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist,

$R^2$ für Wasserstoff steht, oder

für geradkettiges oder verzweigtes Alkyl, Alkenyl, Alkadienyl oder Alkinyl mit jeweils bis zu 10 Kohlenstoffatomen steht, die gegebenenfalls bis zu 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die beiden letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können,

und deren physiologisch unbedenklichen Salze, mit der Maßgabe, daß wenn $R^1$ für m-Nitrophenyl steht, $R^2$ nicht Methyl bedeuten darf.

Verfahren zu ihrer Herstellung und deren Verwendung als Arzneimittel bei der Bekämpfung von Herz-Kreislauferkrankungen, insbesondere des pathologisch veränderten Blutdrucks und der Herzinsuffzienz.

Die vorliegende Erfindung betrifft die Verwendung von 3-Formyl-1,4-dihydropyridinderivaten als Arzneimittel bei der Bekämpfung von Herz-Kreislauferkrankungen, insbesondere des pathologisch veränderten Blutdrucks und der Herzinsuffizienz, neue Verbindungen und Verfahren zu ihrer Herstellung.

Es ist bekannt, daß 1,4-Dihydropyridine mit einer 3,5-Diester-, 3,5-Diacyl- oder 3,5-Ester/Acyl-Gruppierung eine calciumantagonistische Wirkung besitzen [vgl. z.B. DOS 37 11 991].

Außerdem sind N-substituierte 1,4-Dihydropyridinderivate mit einer Formylgruppe in der 3-Position in der EP 330 470 als Chemotherapeutika beschrieben.

In der Publikation [Hons, You Hwa, Suh, Jung Jin, Yuhan Res. Cent., Kunpo 433 - 810, S. Korea, Yakhak Hoechi, 33(5), 290 - 295] wird die Verbindung 5-Formyl-1,4-dihydro-2,6-dimethyl-4-(3-nitro-phenyl)-pyridinmethylester ohne Angabe einer pharmakologischen Wirkung beschrieben.

Die vorliegende Erfindung betrifft 3-Formyl-1,4-dihydropyridine der allgemeinen Formel (I)

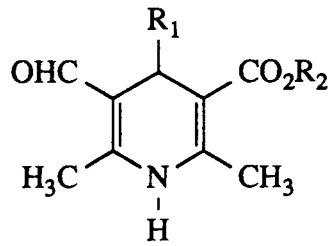

(I)

in welcher

$R^1$ für Phenyl steht, das gegebenenfalls bis zu 3-fach gleich oder verschieden durch Nitro, Cyano, Halogen, Trifluormethyl, Trifluormethylthio, Trifluormethoxy, Difluormethoxy oder durch geradkettiges oder verzweigtes Alkyl, Alkoxy, Alkylthio oder Alkoxycarbonyl mit jeweils bis zu 8 Kohlenstoffatomen,
oder durch eine Gruppe der Formel $-O(CH_2)_n-R^3$, $-S-(CH_2)_n-R^3$ oder $-CO-(CH_2)_n-R^3$ substituiert ist,
worin

n eine Zahl 0, 1, 2, 3 oder 4 bedeutet,

$R^3$ Aryl mit 6 bis 10 Kohlenstoffatomen bedeutet, das gegebenenfalls bis zu 3-fach gleich oder verschieden durch Halogen, Nitro, Trifluormethyl, Trifluormethoxy, Difluormethoxy, Trifluormethylthio oder durch geradkettiges oder verzeigtes Alkyl oder Alkoxy mit jeweils bis zu 8 Kohlenstoffatomen substituiert ist,
oder für einen Rest der Formel

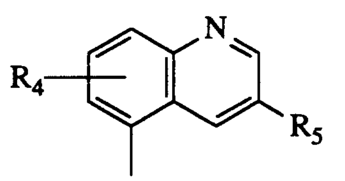

,

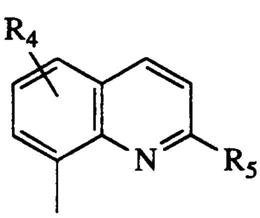

steht,
worin

$R^4$ Wasserstoff, Halogen oder geradkettiges oder verzweigtes Alkyl oder Alkoxy mit jeweils bis zu 8 Kohlenstoffatomen bedeutet,

$R^5$ Phenyl bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist,

$R^2$ für Wasserstoff steht, oder
für geradkettiges oder verzweigtes Alkyl, Alkenyl, Alkadienyl oder Alkinyl mit jeweils bis zu 10 Kohlenstoffatomen steht, die gegebenenfalls bis zu 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die beiden letzteren ihrerseits bis zu 2-fach gleich oder

verschieden durch Halogen oder durch geradkettiges oder verzweigtes Alkyl oder Alkoxy mit jeweils bis zu 6 Kohlenstoffatomen substituiert sein können,

und deren physiologisch unbedenklichen Salze, mit der Maßgabe das wenn $R^1$ für m-Nitrophenyl steht, $R^2$ nicht Methyl bedeuten darf.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),

in welcher

$R^1$ für Phenyl steht, das gegebenenfalls bis zu 2-fach gleich oder verschieden durch Nitro, Cyano, Fluor, Chlor, Trifluormethyl, Trifluormethoxy, Difluormethoxy, durch geradkettiges oder verzweigtes Alkyl, Alkoxy, Alkylthio oder Alkoxycarbonyl mit jeweils bis zu 6 Kohlenstoffatomen, oder durch eine Gruppe der Formel $-O(CH_2)_n-R^3$, $-S(CH_2)_n-R^3$ oder $-CO(CH_2)_n-R^3$ substituiert ist,
worin

n eine Zahl 0, 1 oder 2 bedeutet
und

$R^3$ Phenyl bedeutet, das gegebenenfalls bis zu 2-fach gleich oder verschieden durch Nitro, Fluor, Chlor, Trifluormethyl, Trifluormethoxy, Difluormethoxy oder durch geradkettiges oder verzweigtes Alkyl oder Alkoxy mit jeweils bis zu 6 Kohlenstoffatomen substituiert ist,
oder für einen Rest der Formel

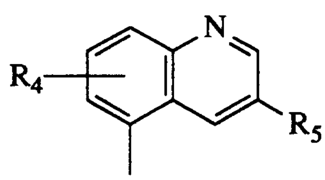

oder

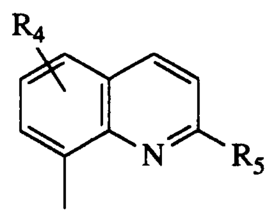

steht,
worin

$R^4$ Wasserstoff bedeutet,

$R^5$ Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist,

$R^2$ für Wasserstoff oder für geradkettiges oder verzweigtes Alkyl oder Alkenyl mit jeweils bis zu 8 Kohlenstoffatomen steht, die gegebenenfalls durch Fluor, Chlor, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 6 Kohlenstoffatomen oder durch Phenoxy oder Phenyl substituiert sind,

mit der Maßgabe, daß wenn $R^1$ für m-Nitrophenyl steht, $R^2$ nicht Methyl bedeuten darf.

Besonders bevorzugt sind Verbindungen der allgemeinen Formel (I), in welcher

$R^1$ für Phenyl steht, das gegebenenfalls durch Nitro, Fluor, Chlor, Brom, Trifluormethyl, Trifluormethoxy, Difluormethoxy, durch geradkettiges oder verzweigtes Alkyl oder Alkoxy mit jeweils bis zu 4 Kohlenstoffatomen oder durch eine Gruppe der Formel $-O(CH_2)_n-R^3$ oder $-S(CH_2)_n-R^3$ substituiert ist,
worin

n die Zahl 1 bedeutet,

$R^3$ Phenyl bedeutet, das gegebenenfalls durch Nitro, Fluor, Chlor, Trifluormethoxy oder durch

geradkettiges oder verzweigtes Alkyl oder Alkoxy mit jeweils bis zu 4 Kohlenstoffaotmen substituiert ist,
oder für einen Rest der Formel

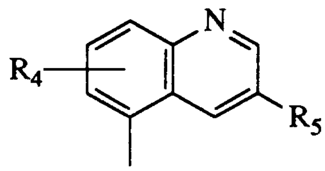

steht,
worin

$R^4$ Wasserstoff bedeutet,

$R^5$ Phenyl bedeutet, das gegebenenfalls durch Fluor, Chlor, Nitro, Trifluormethyl oder durch geradkettiges oder verzweigtes Alkyl oder Alkoxy mit jeweils bis zu 4 Kohlenstoffatomen substituiert ist, bedeutet,

$R^2$ für Wasserstoff steht, oder für geradkettiges oder verzweigtes Alkyl mit bis zu 6 Kohlenstoffatomen steht, das gegebenenfalls durch Hydroxy, Carboxy, Cyano oder durch geradkettiges oder verzweigtes Alkoxycarbonyl, Alkoxy oder Acyloxy mit jeweils bis zu 4 Kohlenstoffatomen substituiert ist

mit der Maßgabe, daß wenn $R^1$ für m-Nitrophenyl steht, $R^2$ nicht Methyl bedeuten darf.

Außerdem wurde ein Verfahren zur Herstellung der erfindungsgemäßen Verbindungen der allgemeinen Formel (I) gefunden, dadurch gekennzeichnet, daß man aktivierte Verbindungen der allgemeinen Formel (II)

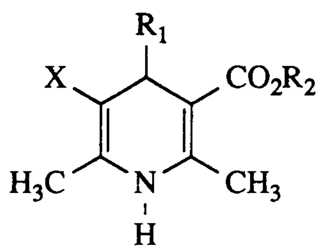
(II)

in welcher

$R^1$ und $R^2$ die oben angegebene Bedeutung haben und

X für einen aktivierenden Rest der Formel

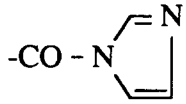
oder
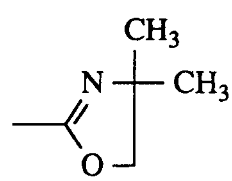

steht,

entweder direkt oder nach einem vorgeschalteten Alkylierungsschritt in inerten organischen Lösemitteln bei Temperaturen von 0-100 ° C reduziert.

Das erfindungsgemäße Verfahren kann durch folgendes Formelschema beispielhaft erläutert werden:

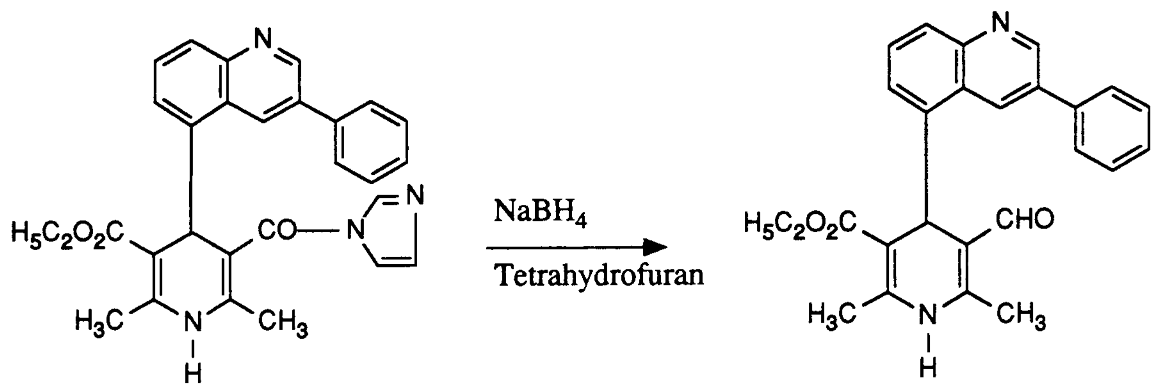


Als Losemittel eignen sich für das Verfahren alle inerten organischen Lösemittel. Hierzu gehören bevorzugt Alkohole wie Methanol, Ethanol, n-bzw. iso-Propanol, Ether wie Diethylether, Tetrahydrofuran, Dioxan oder Glykolmono- oder dimethylether, Eisessig, Pyridin, Dimethylformamid, Dimethylsulfoxid, Acetonitril oder Hexamethylphosphorsäuretriamid oder Acetonitril.

Die Reduktion erfolgt im allgemeinen mit Hydriden wie beispielsweise Natriumborhydrid, Lithiumaluminiumhydrid oder Diisobutylaluminiumhydrid. Bevorzugt ist Natriumborhydrid.

Das Reduktionsmittel wird im allgemeinen in einer Menge von 0,5 mol bis 10 mol, bevorzugt von 1 mol bis 3 mol bezogen auf 1 mol der Verbindungen der allgemeinen Formel (II) eingesetzt.

Die Reduktion erfolgt im allgemeinen in einem Temperaturbereich von 0 ° C bis 100 ° C, bevorzugt von 25 ° C bis 60 ° C.

Die Reduktion kann im allgemeinen bei Normaldruck, erhöhtem oder erniedrigtem Druck (beispielsweise von 0,5 bis 5 bar), vorzugsweise aber bei Normaldruck durchgeführt werden.

Die Alkylierung wird in den oben aufgeführten Lösemitteln bei Temperaturen von 0 ° C bis +150 ° C, vorzugsweise bei Raumtemperatur bis +100 ° C bei Normaldruck durchgeführt.

Als Alkylierungsmittel bei den Verfahren können beispielsweise ($C_1$-$C_8$)-Alkylhalogenide, Sulfonsäureester oder substituierte oder unsubstituierte ($C_1$-$C_6$)-Dialkyl- oder ($C_1$-$C_6$)-Diarylsulfate, vorzugsweise Methyljodid, p-Toluolsulfonsäureester oder Dimethylsulfat eingesetzt werden.

Die vorstehenden Herstellungsverfahren sind lediglich zur Verdeutlichung angegeben. Die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Die Verbindungen der allgemeinen Formel (II) sind zum Teil bekannt oder können nach bekannten Methoden hergestellt werden [vgl. US 4 707 479].

Die erfindungsgemäßen Verbindungen der allgemeinen Formel (I) zeigen ein nicht vorhersehbares, wertvolles pharmakologisches Wirkspektrum. Sie beeinflussen die Kontraktionskraft des Herzens und den Tonus der glatten Muskulatur, insbesondere zeigen sie positiv-inotrope Wirkungen. Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt als Perfusionsmedium dient eine Krebs-Henseleit-Lösung (1) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2$EDTA), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32 ° C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965), 529 - 541). Der Perfusionsdruck wird mittels eines Druckaufnehmers, der vor dem Herzen mit dem

Perfusionssystem in Verbindung steht, registiert. Unter diesen Bedingungen zeigt eine Senkung des Perfusionsdrucks eine Koronardilatation, eine Zu-bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Substanzeffekte auf die Kontraktionsamplitude isolierter Meerschweinchen-Herzvorhöfe beieiner Wirkstoffkonzentration von $10^{-4}$ g/l.

| Bsp.-Nr. | Kontraktionskraft (% Kontrolle) |
|---|---|
| 1 | +32 |
| 3 | +31 |
| 4 | +41 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengenvon etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fallen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ausgangsverbindungen

Beispiel I

1,4-Dihydro-2,6-dimethyl-4-(2-trifluormethyl-phenyl)-3-(4,4-dimethyl-oxazolin-2-yl)-pyridin-5-carbonsäuremethylester

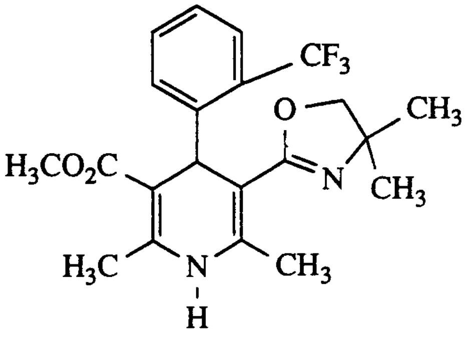

17,4 g (0,1 mol) o-Trifluormethylbenzaldehyd, 15,5 g (0,1 mol) 2-Acetonyl-4,4-dimethyloxazolin und 11,5 g (0,1 mol) β-Aminocrotonsäuremethylester werden in 150 ml i-Propanol 12 h unter Rückfluß erhitzt. Anschließend wird das Lösemittel im Vakuum entfernt und der Rückstand auf einer Kieselgelsäule getrennt. Man erhält 18 g der Titelverbindung.
F°C: 181

Herstellungsbeispiele

Beispiel 1

1,4-Dihydro-2,6-dimethyl-3-formyl-4-(3-phenyl-chinolin-5-yl)-pyridin-5-carbonsäureethylester

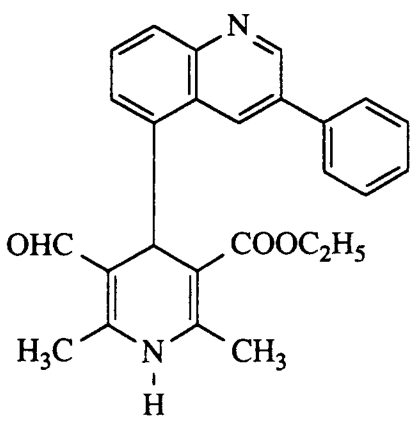

1 g (2,1 mol) 1,4-Dihydro-2,6-dimethyl-4-(3-phenyl-chinolin-5-yl)-pyridin-3-carbonsäureethylester-5-carbonsäureimidazolid werden in 50 ml trockenem Tetrahydrofuran gelöst und mit 500 mg (12,6 mmol) Natriumborhydrid versetzt. Es wird 20 Stunden bei 60°C gerührt, eingeengt, in Essigester gelöst und mit Wasser gewaschen. Die Essigesterphase wird getrocknet und eingeengt. Das Produktgemisch wird über eine Kieselgelsäule mit Toluol/Essigester-Gemischen gereinigt. Man erhält 146 mg einer farblosen Substanz vom Schmelzpunkt 275 - 277°C.

In Analogie zur Vorschrift des Beispiels 1 werden die in Tabelle 1 aufgeführten Beispiele hergestellt:

Tabelle 1:

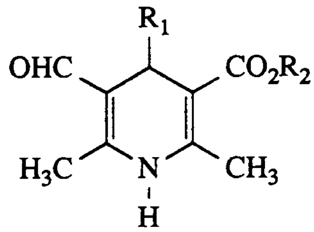

| Bsp.-Nr. | $R^1$ | $R^2$ | F°C |
|---|---|---|---|
| 2 | 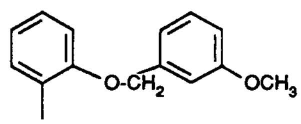 | $-CH_3$ | 186-188 |
| 3 | 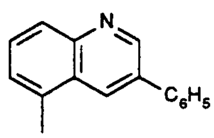 | $-CH(CH_3)_2$ | 261 |
| 4 | 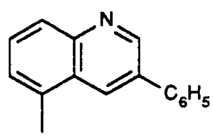 | $-CH_3$ | 282 |
| 5 | 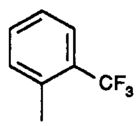 | $-CH_3$ | |

**Patentansprüche**

1. 3-Formyl-1,4-dihydropyridine der allgemeinen Formel (I)

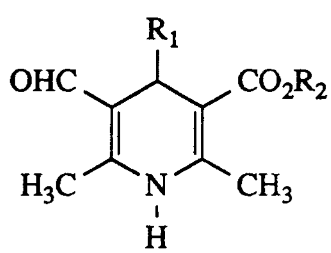

(I)

in welcher

$R^1$ für Phenyl steht, das gegebenenfalls bis zu 3-fach gleich oder verschieden durch Nitro, Cyano, Halogen, Trifluormethyl, Trifluormethylthio, Trifluormethoxy, Difluormethoxy oder durch geradkettiges oder verzweigtes Alkyl, Alkoxy, Alkylthio oder Alkoxycarbonyl mit jeweils bis zu 8 Kohlenstoffatomen, oder durch eine Gruppe der Formel $-O(CH_2)_n-R^3$, $-S-(CH_2)_n-R^3$ oder $-CO-(CH_2)_n-R^3$ substituiert ist,

worin

n eine Zahl 0, 1, 2, 3 oder 4 bedeutet,

$R^3$ Aryl mit 6 bis 10 Kohlenstoffatomen bedeutet, das gegebenenfalls bis zu 3-fach gleich oder verschieden durch Halogen, Nitro, Trifluormethyl, Trifluormethoxy, Difluormethoxy, Trifluormethylthio oder durch geradkettiges oder verzeigtes Alkyl oder Alkoxy mit jeweils bis zu 8 Kohlenstoffatomen substituiert ist,

oder für einen Rest der Formel

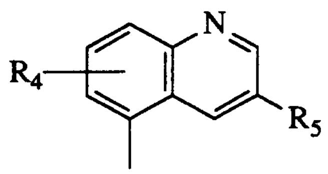
,
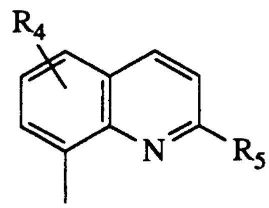

steht,

worin

$R^4$ Wasserstoff, Halogen oder geradkettiges oder verzweigtes Alkyl oder Alkoxy mit jeweils bis zu 8 Kohlenstoffatomen bedeutet,

$R^5$ Phenyl bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist,

$R^2$ für Wasserstoff steht, oder
für geradkettiges oder verzweigtes Alkyl, Alkenyl, Alkadienyl oder Alkinyl mit jeweils bis zu 10 Kohlenstoffatomen steht, die gegebenenfalls bis zu 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die beiden letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können,

und deren physiologisch unbedenklichen Salze, zur Verwendung bei der Bekämpfung von Erkrankungen.

**2.** Verbindungen der allgemeinen Formel (I) gemäß Anspruch 1 mit der Maßgabe das wenn $R^1$ für m-Nitrophenyl steht, $R^2$ nicht Methyl bedeuten darf.

**3.** Verbindungen der allgemeinen Formel (I) gemäß Anspruch 1

in welcher

$R^1$ für Phenyl steht, das gegebenenfalls bis zu 2-fach gleich oder verschieden durch Nitro, Cyano, Fluor, Chlor, Trifluormethyl, Trifluormethoxy, Difluormethoxy, durch geradkettiges oder verzweigtes Alkyl, Alkoxy, Alkylthio oder Alkoxycarbonyl mit jeweils bis zu 6 Kohlenstoffatomen, oder
durch eine Gruppe der Formel $-O(CH_2)_n-R^3$, $-S(CH_2)_n-R^3$ oder $-CO(CH_2)_n-R^3$ substituiert ist,

worin

n eine Zahl 0, 1 oder 2 bedeutet

und

$R^3$ Phenyl bedeutet, das gegebenenfalls bis zu 2-fach gleich oder verschieden durch Nitro, Fluor, Chlor, Trifluormethyl, Trifluormethoxy, Difluormethoxy oder durch geradkettiges oder verzweigtes Alkyl oder Alkoxy mit jeweils bis zu 6 Kohlenstoffatomen substituiert ist,

oder für einen Rest der Formel

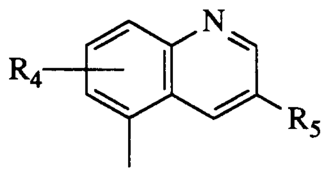

oder

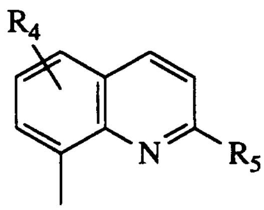

steht,

worin

- $R^4$ Wasserstoff bedeutet,
- $R^5$ Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist,
- $R^2$ für Wasserstoff oder für geradkettiges oder verzweigtes Alkyl oder Alkenyl mit jeweils bis zu 8 Kohlenstoffatomen steht, die gegebenenfalls durch Fluor, Chlor, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 6 Kohlenstoffatomen oder durch Phenoxy oder Phenyl substituiert sind,

mit der Maßgabe, daß wenn $R^1$ für m-Nitrophenyl steht, $R^2$ nicht Methyl bedeuten darf.

**4.** Verbindungen der allgemeinen Formel (I) gemäß Anspruch 1

in welcher

- $R^1$ für Phenyl steht, das gegebenenfalls durch Nitro, Fluor, Chlor, Brom, Trifluormethyl, Trifluormethoxy, Difluormethoxy, durch geradkettiges oder verzweigtes Alkyl oder Alkoxy mit jeweils bis zu 4 Kohlenstoffatomen oder durch eine Gruppe der Formel $-O(CH_2)_n-R^3$ oder $-S(CH_2)_n-R^3$ substituiert ist,

worin

- n die Zahl 1 bedeutet,
- $R^3$ Phenyl bedeutet, das gegebenenfalls durch Nitro, Fluor, Chlor, Trifluormethoxy oder durch geradkettiges oder verzweigtes Alkyl oder Alkoxy mit jeweils bis zu 4 Kohlenstoffatomen substituiert ist,

oder für einen Rest der Formel

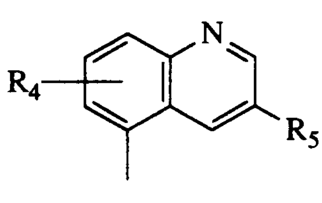

steht,

worin

- $R^4$ Wasserstoff bedeutet,
- $R^5$ Phenyl bedeutet, das gegebenenfalls durch Fluor, Chlor, Nitro, Trifluormethyl oder durch geradkettiges oder verzweigtes Alkyl oder Alkoxy mit jeweils bis zu 4 Kohlenstoffatomen substituiert ist, bedeutet,
- $R^2$ für Wasserstoff steht, oder für geradkettiges oder verzweigtes Alkyl mit bis zu 6 Kohlenstoffatomen steht, das gegebenenfalls durch Hydroxy, Carboxy, Cyano oder durch geradkettiges oder verzweigtes Alkoxycarbonyl, Alkoxy oder Acyloxy mit jeweils bis zu 4 Kohlenstoffatomen substituiert ist

mit der Maßgabe, daß wenn $R^1$ für m-Nitrophenyl steht, $R^2$ nicht Methyl bedeuten darf.

**5.** Verfahren zur Herstellung von Verbindungen der allgemeinen Formel (I)

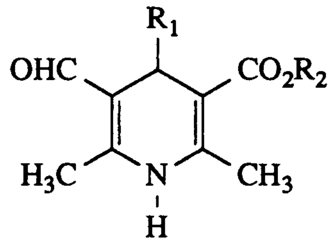

(I)

in welcher

$R^1$ für Phenyl steht, das gegebenenfalls bis zu 3-fach gleich oder verschieden durch Nitro, Cyano, Halogen, Trifluormethyl, Trifluormethylthio, Trifluormethoxy, Difluormethoxy oder durch geradkettiges oder verzweigtes Alkyl, Alkoxy, Alkylthio oder Alkoxycarbonyl mit jeweils bis zu 8 Kohlenstoffatomen, oder durch eine Gruppe der Formel $-O(CH_2)_n-R^3$, $-S-(CH_2)_n-R^3$ oder $-CO-(CH_2)_n-R^3$ substituiert ist,

worin

n eine Zahl 0, 1, 2, 3 oder 4 bedeutet,

$R^3$ Aryl mit 6 bis 10 Kohlenstoffatomen bedeutet, das gegebenenfalls bis zu 3-fach gleich oder verschieden durch Halogen, Nitro, Trifluormethyl, Trifluormethoxy, Difluormethoxy, Trifluormethylthio oder durch geradkettiges oder verzeigtes Alkyl oder Alkoxy mit jeweils bis zu 8 Kohlenstoffatomen substituiert ist,

oder für einen Rest der Formel

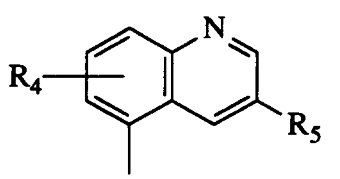 , 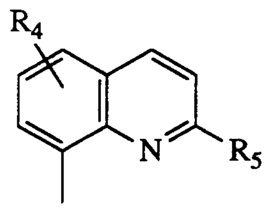

steht,

worin

$R^4$ Wasserstoff, Halogen oder geradkettiges oder verzweigtes Alkyl oder Alkoxy mit jeweils bis zu 8 Kohlenstoffatomen bedeutet,

$R^5$ Phenyl bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist,

$R^2$ für Wasserstoff steht, oder

für geradkettiges oder verzweigtes Alkyl, Alkenyl, Alkadienyl oder Alkinyl mit jeweils bis zu 10 Kohlenstoffatomen steht, die gegebenenfalls bis zu 2-fach gleich oder verschieden durch Halogen, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die beiden letzteren ihrerseits bis zu 2-fach gleich oder verschieden durch Halogen oder durch geradkettiges oder verzweigtes Alkyl

oder Alkoxy mit jeweils bis zu 6 Kohlenstoffatomen substituiert sein können, und deren physiologisch unbedenklichen Salze, mit der Maßgabe das wenn $R^1$ für m-Nitrophenyl steht, $R^2$ nicht Methyl bedeuten darf,
dadurch gekennzeichnet, daß man aktivierte Verbindungen der allgemeinen Formel (II)

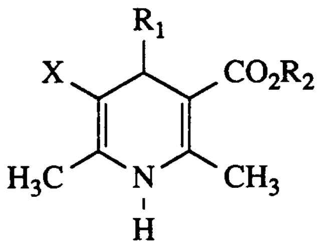

(II)

in welcher

$R^1$ und $R^2$ die oben angegebene Bedeutung haben

und

X für einen aktivierenden Rest der Formel

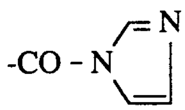 oder 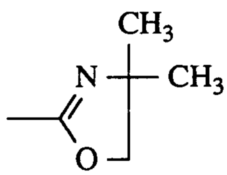

steht,

entweder direkt oder nach einem vorgeschalteten Alkylierungsschritt in inerten organischen Lösemitteln bei Temperaturen von 0-100 ° C reduziert.

6. Arzneimittel enthaltend mindestens eine Verbindung der allgemeinen Formel (I) gemäß Anspruch 1.

7. Verfahren zur Herstellung von Arzneimitteln dadurch gekennzeichnet, daß man mindestens eine Verbindung der allgemeinen Formel (I) gemäß Anspruch 1 gegebenenfalls unter Verwendung von üblichen Hilfs- und Trägerstoffen in eine geeignete Applikationsform überführt.

8. Verwendung von Verbindungen der allgemeinen Formel I gemäß Anspruch 1 bei der Herstellung von Arzneimitteln zur Behandlung von Herz-Kreislauferkrankungen.

Europäisches Patentamt

# EUROPÄISCHER RECHERCHENBERICHT

Nummer der Anmeldung

EP 92 10 8736
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | CHEMICAL ABSTRACTS, vol. 114, no. 5, 4. Februar 1991, Columbus, Ohio, US; abstract no. 42505W, Y.H. HONG ET AL.: 'SYNTHESIS OF 1,4-DIHYDROPYRIDINE-5-FORMYL DERIVATIVES' Seite 718 ; Spalte 1 ; * Zusammenfassung * | 2-5 | C07D211/90<br>C07D401/04<br>A61K31/44<br>//C07D401/06<br>C07D413/04<br>C07D401/14<br>C07D413/14 |
| A | JOURNAL OF THE CHEMICAL SOCIETY, CHEMICAL COMMUNICATIONS Nr. 12, 15. Juni 1986, Seiten 920 - 921; A.I. MEYERS AND T. OPPENLAENDER: 'AN ASYMMETRIC SYNTHESIS OF CHIRAL NIFEDIPINE ANALOGUES' * Seite 921; Beispiele 2,6,7 * | 1-8 | |
| D,A | DE-A-3 711 991 (BAYER AG) 20. Oktober 1988<br>* das ganze Dokument *<br>* Seite 16, Zeile 36 - Seite 17, Zeile 3 * Verfahrensvariante H<br>* Seite 15 * | 1-8<br><br>5 | |
| | | | **RECHERCHIERTE SACHGEBIETE (Int. Cl.5)** |
| D,A | EP-A-0 330 470 (AJINOMOTO CO., INC. AND JAPANESE FOUNDATION FOR CANCER RESEARCH) 30. August 1989<br>* das ganze Dokument * | 2-4 | C07D<br>A61K |
| D,A | US-A-4 707 479 (BAYER AG) 17. November 1987<br>* das ganze Dokument *<br>* Spalte 8, Zeile 63 - Spalte 9, Zeile 59 * | 1-8<br><br>5 | |
| A | WO-A-8 602 640 (BRISTOL-MYERS COMPANY) 9. Mai 1986<br>* das ganze Dokument *<br>Zwischenprodukte der Formel (XIIC)<br>* Seite 20; Tabelle 2 * | 1-8<br><br>5 | |

-/--

Der vorliegende Recherchenbericht wurde für alle Patentansprüche erstellt

| Recherchenort | Abschlußdatum der Recherche | Prüfer |
|---|---|---|
| MUENCHEN | 16 SEPTEMBER 1992 | HARTRAMPF G.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

EP 92 10 8736
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | US-A-4 414 213 (MEAD JOHNSON & COMPANY) 8. November 1983<br>* das ganze Dokument *<br>* Spalte 9, Zeile 40 - Spalte 16, Zeile 40; Beispiel 51; Tabelle 4 *<br>--- | 1-8<br><br>5 | |
| P,A | EP-A-0 452 712 (BAYER AG) 23. Oktober 1991<br>* das ganze Dokument *<br>----- | 1-8 | |

RECHERCHIERTE SACHGEBIETE (Int. Cl.5 )

Der vorliegende Recherchenbericht wurde für alle Patentansprüche erstellt

| Recherchenort | Abschlußdatum der Recherche | Prüfer |
|---|---|---|
| MUENCHEN | 16 SEPTEMBER 1992 | HARTRAMPF G.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)